# EUROPÄISCHE PATENTSCHRIFT

(11) **EP 0 776 668 B1**
(45) Veröffentlichungstag und Bekanntmachung des Hinweises auf die Patenterteilung: **24.09.2003**
(21) Anmeldenummer: 96117241.8
(22) Anmeldetag: 28.10.1996
(51) Int. Cl.: A61L 2/18, B01D 15/00

(54) **Verfahren zur Entfernung von aromatischen Verbindungen aus produkthaltigen Lösungen**
Process for the removal of aromatic compounds from solutions
Procédé pour éliminer les composés aromatiques des solutions

(30) Priorität: 28.11.1995 DE 19544297
(43) Veröffentlichungstag der Anmeldung: 04.06.1997
(73) Patentinhaber: Aventis Behring Gesellschaft mit beschränkter Haftung, 35002 Marburg (DE)
(72) Erfinder: Schüler, Eckhard, Dr., 35041 Marburg (DE); Wenz, Karl-Heinz, 35096 Weimar (DE)

(56) Entgegenhaltungen:
- EP-A- 0 312 248
- EP-A- 0 366 946
- WO-A-95/00631
- WO-A-95/16348
- WO-A-96/40857
- US-A- 4 673 733
- MARGOLIS-NUNNO H ET AL: "Elimination of potential mutagenicity in platelet concentrates that are virally inactivated with psoralens and ultraviolet A light" TRANSFUSION, Bd. 35, Nr. 10, Oktober 1995 (1995-10), Seiten 855-862, XP000892781
- HOROWITZ B ET AL: "Solvent/detergent-treated plasma: a virus-inactivated substitute for fresh frozen plasma" BLOOD, Bd. 79, Nr. 3, Februar 1992 (1992-02), Seiten 826-831, XP000905211

## Beschreibung

Die vorliegende Erfindung betrifft ein Verfahren zur Entfernung von aromatischen Verbindungen aus produkthaltigen Lösungen.

Aromatische Verbindungen wie beispielsweise Acridin oder Acridinderivate finden schon seit langem Anwendung in der Medizin, so z.B. bei der Wundbehandlung. Es ist darüber hinaus bekannt, daß mit Hilfe von Acridin und Acridinderivaten sowohl hüllhaltige Viren als auch nicht-umhüllte Viren wie z.B. das Poliovirus inaktiviert werden können. Hierbei ist es unter Umständen sehr wichtig, diese nach ihrer Anwendung für die Virusinaktivierung wieder zu entfernen, so daß ihre Konzentration im Endprodukt auch bei einer Langzeitbehandlung toxikologisch unbedenklich bleibt.

Eine einfache Methode für die Entfernung von Acridinen aus einer Lösung besteht in ihrer Aussalzung. So wurde z. B. Rivanol® bei verschiedenen Proteinreinigungsverfahren durch Aussalzen vom Produkt abgetrennt. Dieses Vorgehen hat jedoch bezüglich einer eventuellen Anwendung auf Plasmaproteine diverse Nachteile: Zum einen kann ein Teil des Proteins und damit des Produktes mitgefällt werden, was zu Ausbeuteverlusten führt. Zum anderen ist die Abtrennung von Acridin bzw. seinen Derivaten bei einer solchen Vorgehensweise nach diesem einen Schritt nicht vollständig. Bei einem pharmazeutischen Produktionsverfahren sollte ein zugeführtes Agens nach seiner Verwendung aber wieder so stark abgereichert werden können, daß nur noch wenige ppm als ungebundene Form im Produkt enthalten sind. Dies ist vor allem bei der Herstellung von Substanzen, die z.B. im Rahmen einer Langzeittherapie mehrfach appliziert werden sollen, wichtig.

Die Abtrennung nicht-denaturierender, amphiphiler Substanzen aus Plasmaproteinprodukten durch Fällung der Plasmaproteine wird in EP-A-0 050 061 offenbart. Ein Ausfällen ist vielfach aber wie oben beschrieben nicht zweckmäßig, um ein hochreines Produkt zu erhalten.

Ein Verfahren zur Entfernung von lipidlöslichen Verfahrenschemikalien aus einem biologischen Material durch hydrophobe Austauschchromatographie an einem C-6 bis C-24 Harz wird in der europäischen Patentschrift Nr. 0 366 946 offenbart.

Ein ähnliches Verfahren, das in einem "continous-flow" System zur Bearbeitung von Blut oder Blutprodukten verwendet werden kann, wird in der WO 95/00631 beschrieben.

In der EP 0 312 248 wird ein Verfahren zur Aufreinigung von Acridiniumderivaten u. a. mittels "reversed phase" Chromatographie beschrieben.

Eine Aufgabe der vorliegenden Erfindung ist es somit ein Verfahren zur Verfügung zu stellen, mit dem aromatische Verbindungen aus produkthaltigen, insbesondere proteinhaltigen Lösungen entfernt werden können, ohne dass gleichzeitig signifikante Änderungen der Produktzusammensetzung oder der Struktur der einzelnen Komponenten auftreten. Eine vorhandene biologische Aktivität der Lösung soll dabei weitgehend erhalten bleiben.

Eine weitere Aufgabe ist darin zu sehen, das Verfahren so auszugestalten, dass auch an die Produkte gebundene aromatische Verbindungen aus der Lösung entfernt werden können, bzw. dass die Bindung von aromatischen Verbindungen an die Produkte verhindert oder zumindest gehemmt wird.

Es wurde nun gefunden, dass sich aromatische Verbindungen aus einer wässrigen Lösung, die Proteine oder Peptide mit biologischer Aktivität enthält, durch In-Kontakt-bringen der Lösung mit einem Trägermaterial entfernen lassen, wobei das Trägermaterial durch C₁ bis C₅-Alkylreste modifiziert ist und die protein- oder peptidhaltige Lösung von dem Trägermaterial anschließend getrennt wird.

Bevorzugt werden Acridin, Hypericin, Psoralen, Methylenblau und Derivate dieser Verbindungen aus der Lösung entfernt. Insbesondere bevorzugt wird Acridin und/oder ein Acridinderivat aus der Lösung entfernt.

Acridinderivate sind beispielsweise Ethacridin, 9-Aminoacridin (=Aminacrin), 3,6-Acridindiamin (Proflavin), Acrisorcin, Acrizanchlorid (=Phenacridanchlorid), Acridinorange, Quinacrin, Acricid, Acridon, Acridin-9-carbonsäure, Acranil (1-[(6-Chloro-2-methoxy-9-acrinidyl)-amino]-3-(diethylamin)-2-propanol-dihydrochlorid), 3,7-Diamino-5-phenylphenaziniumchlorid (Phenosafranin, Safranin B extra), Phenoxazin, Phenothiazin und Acriflavin (3,6-Diamino-1 0-methylacridiniumchlorid), sowie deren Salze wie z. B. Chloride, Sulfate und Bromide. Bevorzugte Acridinderivate sind Proflavin und Acriflavin.

Als produkthaltige Lösung wird bei dem erfindungsgemäßen Verfahren insbesondere eine Proteinlösung oder eine Peptidlösung bevorzugt. Ebenfalls bevorzugt werden wässrige Lösungen eingesetzt. Die Lösung kann beispielsweise folgende Ausgangsmaterialien umfassen:

Blutplasma, Blutserum, Cryopräzipitat, Fibrinogen, Thrombin, Prothrombin, Faktor V, Faktor VII, Faktor VIII, Faktor IX, Faktor X, Faktor XII, Faktor XIII. Antithrombin III, Albumin, Präalbumin, α1-Globuline, β-Globuline, Gamma-Globuline, Immunglobulin G, Immunglopulin M, Immunglobulin A, Immunglobulin D, Immunglobulin E, Fibrinogen, Fibronectin, α1-Antiplasmin, Antitrypsin, Plasminogen, Plasmin, Urokinase, Prourokinase, PAI-1, PAI-2, Protein C, Protein S, Protein C-Inhibitor, lyophilisiertes Serum, Fraktion 1, Fraktion II, Fraktion III, Fraktion IV, Fraktion IV-1, Fraktion IV-2, Fraktion IV-3, Fraktion IV-4, Fraktion V, Fraktion VI, Retinol-bindendes Protein, Transferrin, Kininogen, Gewebeplasminogenaktivator, Kallikrein, Thrombomodulin, Vitronektin, GC-Globulin, Makroglobulin, Leukozytenproteinaseinhibitor, Wachstumsfaktoren, Zellkulturüberstand von normalen und permanenten Zellen, Extrakte aus normalen und permanenten Zellen, Vollblut, Körperflüssigkeiten, Zellsuspensionen, Zellkulturüberstände, Extrakte aus Hybridomen, Extrakte aus tierischen oder pflanzlichen Geweben, Extrakte oder Überstände von Mikroorganismen, Sekrete transgener Zellen bzw. Tiere, Kulturlösungen, Extrakte oder Suspensionen von oder Suspensionen von Pflanzenzellen, Organextrakte, Milch, Antigenpräparationen oder Vaccine.

Bekannte polar modifizierte Gel-Trägermaterialien werden unter den Handelsnamen LiChroprep CN®, LiChroprep NH₂® und LiChroprep Diol® von der Firma Merck (Darmstadt) vertrieben. Zu diesen Trägermaterialien gehören auch Mischpolymerisate wie das von Biosepra unter dem Handelsnamen SDR Hyper D®-Gel vertriebene Produkt.

Um einen Ausbeuteverlust des Produkts so gering wie möglich zu halten, werden hydrophob modifizierte Trägermaterialien mit kurzen Seitengruppen verwendet, da diese eine geringe Affinität zu Proteinen aufweisen. Unter kurzen Seitengruppen werden hier solche Gruppen verstanden, die 1 bis 5 Kohlenstoffatome aufweisen, insbesondere C₁-C₅-Alkylreste. Besonders bevorzugt werden Kieselgele, die mit Ethylgruppen hydrophob modifiziert sind.

In diese Kategorie von Trägermaterialien fallen z. B. folgende Handelsprodukte: LiChroprep RP-2 (Fa. Merck, Darmstadt), silanisiertes Kieselgel 60 (Fa. Merck, Darmstadt) und TMS-250 (C₁-Alkylrest, end-capped mit Trimethylsilyl-Gruppen; Fa. TosoHaas).

Um die hydrophile Wechselwirkung des Produktes mit dem Trägermaterial und damit Ausbeuteverluste zu reduzieren, ist es von Vorteil, hierfür die geladenen Gruppen des Trägermaterials zu blockieren, z. B. mit Dimethylsilyl- oder Trimethysilylgruppen. In diese Gruppe von Trägermaterialien fallen z. B. ODS-Prep (C18, end-capped. Fa. TosoHaas) und Prep C18 (von Fa. Waters).

Erfindungsgemäß kann das In-Kontakt-bringen der Lösung mit dem Trägermaterial durch Säulenchromatographie oder Batch-Chromatographie erfolgen. Weiterhin besteht die Möglichkeit, die Lösung durch Filtration durch das Trägermaterial oder Überströmen des Trägermaterials mit diesem in Kontakt zu bringen. Dabei lässt man die Lösung durch oder über eine geeignete Vorrichtung, beispielsweise eine Membran, die das Trägermaterial enthält oder mit diesem beschichtet ist, strömen. Beispielsweise eignet sich eine Membran, an der die funktionelle Gruppe, insbesondere eine hydrophobe oder geladene Gruppe, immobilisiert ist. Bevorzugt sollten aromatische Verbindungen aus einer Lösung, die bis zu 50 mg/ml dieser Substanzen enthält, entfemt werden. Der Restgehalt an aromatischen Verbindungen in der Lösung nach dem In-Kontakt-bringen mit dem Trägermaterial kann dann mittels Photometrie, z. B. Fluoreszenzphotometrie, bestimmt werden. Bevorzugt liegt der Restgehalt bei weniger als 1 ppm, besonders bevorzugt bei weniger als 0,1 ppm.

Der Restgehalt an aromatischen Verbindungen, der nach In-Kontakt-bringen mit dem Trägermaterial in der Lösung verbleibt, ist u. a. auf die Bindung von aromatischen Verbindungen an das Produkt, insbesondere an Proteine, zurückzuführen. Dieser Restgehalt kann erfindungsgemäß dadurch weiter gesenkt werden, dass der Lösung vor dem In-Kontakt-bringen mit dem Trägermaterial ein Additiv zugesetzt wird, welches die Bindung von aromatischen Verbindungen an Proteine weitgehend verhindert oder bereits gebundene aromatische Verbindungen wieder löst.

Als Additive eignen sich dabei hydrophobe und amphiphile Substanzen wie Detergentien, die der Lösungen in einer Konzentration von 0,1-5 %, vorzugsweise von 0,2 - 1,5 % ganz bevorzugt von 0,3 - 1,2 % zugegeben werden können.

Geht der Entfernung von aromatischen Verbindungen eine Virusinaktivierung durch Acridin oder Acridinderivate voraus, so ergeben sich zwei Möglichkeiten für den Additivzusatz. Entweder die Bindung von Acridin und Acridinderivaten an die Proteine wird schon bei der Virusinaktivierung durch Acridin und Acridinderivate reduziert, dadurch, dass die hydrophoben Domänen der Proteine durch die Zugabe von hydrophoben bzw. amphiphilen Substanzen blockiert werden, oder das bereits gebundene Acridin bzw. Acridinderivat wird nach der Virusinaktivierung durch eine hydrophobe Substanz bzw. durch ein Detergens verdrängt. Der Lösungsansatz, das Protein-gebundene Acridin oder Acridinderivat erst nach einer Virusinaktivierung wieder vom Produkt abzulösen, ist vor allem dann interessant, wenn der Zusatz einer konkreten amphiphilen Substanz bzw. eines Detergens mit der Virusinaktivierungsmethode negativ interferieren würde. In diesem Fall ist die Zugabe eines Additivs zum Zwecke der Ablösung von Protein-gebundenem Acridin und Acridinderivaten erst nach der eigentlichen Virusinaktivierung durch Acricin oder Acridinderivate möglich.

Bevorzugt wird das Additiv gleichzeitig mit der aromatischen Verbindung aus der Lösung entfernt. Besonders bevorzugt ist das erfindungsgemäße Verfahren, bei dem die gleichzeitige Entfernung von Acridin und/oder Acridinderivaten und Detergentien, z. B. Triton X-100, mit Hilfe einer Reversed-Phase-Chromatographie durch Bindung dieser Substanzen an ein Silicia-Trägermaterial, an das C₁- bis C₂₄-Seitengruppen gebunden sind, erfolgt.

Als Additive eignen sich beispielsweise Polyvinylalkohole (PVA), Polyvinylpyrrolidone, Tween® 20, Tween® 40, Tween® 60, Tween® 80, Nonidet® P40, Polyethylenglycole, Deoxycholat, CHAPS, oxyethyliertes Alkylphenol (z. B. Triton® X-100), partiell mit Fettsäure verestertes Polyoxyethylen (z. B. Myrj 45), Polyoxyethylen-Fettalkoholderivate (z. B. Brij), Dodecyl-N-betain, Palmitoyllysolecithin, Dodecyl-β-alanin, N-Dodecylaminoethansulfonsäure, Tetradecylammoniumbromid, Hexadecyltrimethylammoniumchlorid, Dodecyl-pyrimidiniumchlorid, Ethylenoxid-Propalenoxid Kondensate (PluronicBlockcopolymere), Cetyltrimethylammoniumbromid, Dodecyltri-methylbenzylammoniumchlorid (Triton® K-60), oxyethylierte Amine (Ethomeen), sulfatiertes oxyethyliertes Alkylphenol (Triton® W-30), Natriumcholat, Natriumdesoxycholat, Igepon A (Natriumsulfoethylsulfonat), Igepon T (N-Methyl-N-oleylethanol-sulfonat) und Nacconol ® NR (Natrium-dodecylbenzensulfonat).

Die chemische Bezeichnung der verwendeten Markennamen ist im Folgenden aufgeführt, sofern nicht schon oben genannt:
- Tween® 20:}: Poly(oxyethylene)n-sorbitan-monolaureat
- Tween® 40:}: Poly(oxyethylene)n-sorbitan-monopalmitat
- Tween® 60:}: Poly(oxyethylene)n-sorbitan-monostearat
- Tween® 80: }: Poly(oxyethylene)n-sorbitan-monooleat
- Nonidet® P40:: Ethylphenolpoly(ethylen)nglycolether
- CHAPS:: 3-[(3-Cholamidopropyl)dimethylammonio]-1 -propansulfonat
- Triton® X-100:: Octylphenolpoly(ethylenglycolether)n

In einer weiteren Ausführungsform schließt sich das erfindungsgemäße Verfahren einem Verfahren zur Inaktivierung von Viren durch Acridin und/oder Acridinderivate an. Beispielsweise kann ein Verfahren zur Herstellung von Virus-inaktiviertem Blutplasma die Zugabe von Acridin und/oder Acridinderivaten zu dem Blutplasma, eine Inkubation des Acridins und/oder Acridinderivats mit dem Blutplasma und die wie oben beschriebene Entfernung des Acridins und/oder der Acridinderivate aus dem Blutplasma umfassen. Das und/oder die Acridinderivate werden dabei bevorzugt in einer Menge von 10 ug bis100 mg pro Liter zugesetzt. Die Inkubation kann für 1 - 6 Stunden erfolgen und das gesamte Verfahren wird bevorzugt bei einer Temperatur von 2°C bis 50°C, insbesondere bei 10°C bis 37°C oder 10°C bis 25 °C durchgeführt.

In einer weiteren Ausführungsform dieser Erfindung wird in dem Verfahren zur Virus-Inaktivierung nach dem Inkubationsschritt ein Additiv zugesetzt, welches die Bindung von Acridin und Acridinderivaten an Proteine weitgehend verhindert bzw. bereits gebundenes Acridin und Acridinderivate wieder löst. Hierzu werden bevorzugt Detergentien wie Triton X-100 oder Triton X-114 in einer Konzentration von bis zu 1 %, vorzugsweise 0,2 % - 0,8 %, ganz bevorzugt 0,4 % - 0,7 % zugesetzt. Vorzugsweise werden anschließend das Acridin und/oder die Acridinderivate zusammen mit dem Additiv über eine Chromatographiesäule mit hydrophobem Trägermaterial, insbesondere einem C₂-Trägermaterial, z. B. eine LiChroprep-RP-Säule, in einem Schritt abgetrennt.

In dieser Ausführungsform ist es weiterhin bevorzugt, die Virus-inaktivierte Lösung mit Triton X-100 zu versetzen und für 5 - 60 Minuten, vorzugsweise für 5 - 30 Minuten z. B. unter Rühren zu inkubieren und dann über eine Chromatographiesäule sowohl das Acridin und/oder die Acridinderivate als auch das Triton X-100 zu entfernen.

Das erfindungsgemäße Verfahren hat den Vorteil, dass aromatische Verbindungen aus einer produkthaltigen Lösung, insbesondere einer Proteinlösung, ohne gleichzeitige signifikante Veränderung der Produktzusammensetzung oder einer Veränderung der biologischen Aktivität dieser Lösung entfernt werden können. Weiterhin ergibt sich aus einer Kombination eines Verfahrens zur Virus-Inaktivierung mit Acridin und/oder Acridinderivaten mit dem erfindungsgemäßen Verfahren zur Entfernung dieser Substanzen aus der Lösung der Vorteil, dass noch bei der Chromatographie von Virus-haltigem Material weitere vorher noch nicht durch Acridin bzw. Acridinderivate inaktivierte Viren (z. B. Nicht-umhüllte Viren wie z. B. Parvoviren oder umhüllte Viren wie z. B. BVDV-Viren) von dem Trägermaterial zurückgehalten und damit aus der Produktlösung entfernt werden, wodurch ein Beitrag zur Gesamtvirusabreicherung eines Produktionsverfahrens beigesteuert wird.

Ein weiterer Vorteil ergibt sich bei dem Einsatz von C₂-hydrophob modifizierten Trägermaterialien, da die Bindung von aromatischen Verbindungen aus wässriger Lösung schnell und effektiv verläuft, so gut wie kein hydrophobes Material (z. B. Proteine nicht-viralen Ursprungs) gebunden wird und dadurch eventuelle Produktverluste minimal gehalten werden. Dies erlaubt z. B. die schnelle Entfernung von aromatischen Verbindungen aus Virus-haltigen Proteinlösungen mit sehr hohen Protein- und Aktivitätsausbeuten.

Die folgenden Beispiele sollen die vorliegende Erfindung näher erläutern. Mit Ausnahme der Angaben für die Acriflavin- bzw. Proflavinkonzentration - diese sind in Gew.-% angegeben - beziehen sich die Konzentrationsangaben auf Vol. %.

### Beispiel 1

### Entfernung von 0,001% Acriflavin aus einer Faktor VIII-enthaltenden Lösung mit LiChroprep RP-2

In diesem Beispiel und in den folgenden Beispielen sind mit Ausnahme der Angaben für die Acriflavin - bzw. Proflavinkonzentration - diese sind in Gewichtsprozenten angegeben - die Konzentrationsangaben in Volumenprozent angegeben, so z. B. die Angaben für PVA und Triton in den Beispielen 2 und 3.

20 ml einer Lösung, die Faktor F VIII und von Willebrand-Faktor mit einer OD₂₈₀-320 von 2,5 und einem pH-Wert von 6,9 enthält, wird 0,001% Acriflavin zugemischt. Anschließend wird der Ansatz über eine Säule, die 4 ml LiChroprep RP-2 enthält, gepumpt. Der Durchlauf enthält nur noch 0,2 ppm Acriflavin. Die Bestimmung des Acriflavins erfolgt photometrisch bei 450 nm.

### Beispiel 2

### Entfernung von 0,001% Acriflavin in Gegenwart von Polyvinylalkohol oder von Polyvinylpyrrolidon aus einer Faktor VIII-enthaltenden Lösung mit LiChroprep RP-2

20 ml einer Lösung, die Faktor F VIII und von Willebrand-Faktor mit einer OD₂ₛₒ-320 von 2,5 und einem pH-Wert von 6,9 enthält, wird Acriflavin bis zu einer Endkonzentration von 0,001% und Polyvinylalkohol PVA (Hoechst AG) oder Polyvinylpyrrolidon PVP K60 (BASF), in verschiedenen Konzentrationen zugemischt. Anschließend wird der Ansatz über eine Säule, die 4 ml LiChroprep RP-2 enthält, gepumpt. Anschließend wird der Gehalt an Acriflavin in der Proteinlösung photometrisch bestimmt. Wie aus nachstehender Tabelle 1 zu entnehmen ist, läßt sich sowohl mit PVA als auch mit PVP die unspezifische Bindung von Acriflavin an das Protein konzentrationsabhängig verhindern.

**Tabelle 1:**

| Einfluß von PVA und PVP auf die im Produkt verbleibende Restkonzentration an Acriflavin | | |
|---|---|---|
| Additiv | Konzentration im Ansatz [%] | Acriflavin-Restkonzentration |
| PVA | 0,1% | 0,3 ppm |
| PVA | 1,0% | < 0,1 ppm^{*}) |
| PVP | 2,0% | < 0,1 ppm^{*}) |

| | | |
|---|---|---|
| ^{*}) Die photometrische Nachweisgrenze liegt bei 0,1 ppm. | | |

### Beispiel 3

### Entfernung von 0,001% Acriflavin aus einer Faktor VIII-enthaltenden Lösung nach Zusatz von Triton X-100 mit Hilfe von LiChroprep RP-2

20 ml einer Lösung, die Faktor F VIII und von Willebrand-Faktor mit einer 0D₂₈₀₋₃₂₀ von 2,5 und einem pH-Wert von 6,9 enthält, wird Acriflavin bis zu einer Endkonzentration von 0,001% zugemischt. Nach einer Inkubationszeit von 4 Stunden wird dem Ansatz noch Triton X-100 in verschiedenen Konzentrationen zugemischt und nach 10 Minuten Rühren wird der Ansatz über eine Säule, die 4 ml LiChroprep RP-2 enthält, gepumpt. Anschließend wird der Gehalt an Acriflavin in der Proteinlösung photometrisch bestimmt. Wie aus nachstehender Tabelle 2 zu entnehmen ist, läßt sich mit Triton X-100 das Acriflavin aus seiner unspezifischen Bindung an das Protein in konzentrationsabhängiger Weise verdrängen.

**Tabelle 2:**

| Einfluß von Triton X-100 auf die im Produkt verbleibende Restkonzentration an Acriflavin | | |
|---|---|---|
| Additiv | Konzentration im Ansatz [%] | Acriflavin-Restkonzentration |
| Triton X-100 | 0,1% | 0,4 ppm |
| Triton X-100 | 0,2% | 0,2 ppm |
| Triton X-100 | 0,4% | < 0,1 ppm ^{*}) |
| Triton X-100 | 0,5% | < 0,1 ppm ^{*}) |

| | | |
|---|---|---|
| *) Die photometrische Nachweisgrenze liegt bei 0,1 ppm. | | |

## Patentansprüche

1. Ein Verfahren zur Entfernung von aromatischen Verbindungen aus einer wässrigen Lösung, die Proteine oder Peptide mit biologischer Aktivität enthält, wobei diese Lösung mit einem Trägermaterial in Kontakt gebracht wird, das durch C₁ bis C₅ Alkylreste modifiziert ist und anschließend die Protein- oder Peptidhaltige Lösung von dem modifizierten Trägermaterial getrennt wird.

2. Verfahren nach Anspruch 1, **dadurch gekennzeichnet, dass** die aromatische Verbindung ausgewählt ist aus der Gruppe bestehend aus: Acridin, Hypericin, Psoralen, Methylenblau und Derivaten dieser Verbindungen.

3. Verfahren nach Anspruch 1, **dadurch gekennzeichnet, dass** die aromatische Verbindung Acridin und/ oder ein Acridinderivat ist.

4. Verfahren nach mindestens einem der Ansprüche 1 bis 3, **dadurch gekennzeichnet, dass** das Trägermaterial ein Kieselgel ist.

5. Verfahren nach Anspruch 4, **dadurch gekennzeichnet, dass** das Kieselgel mit Ethylgruppen hydrophob modifiziert ist.

6. Verfahren nach einem der Ansprüche 1 bis 5, **dadurch gekennzeichnet, dass** das Verfahren durch Säulenchromatographie oder Batch-Chromatographie ausgeführt wird. .

7. Verfahren nach einem der Ansprüche 1 bis 5, **dadurch gekennzeichnet, dass** das Verfahren durch Filtration durch das Trägermaterial oder Überströmen des Trägermaterials ausgeführt wird.

8. Verfahren nach einem der Ansprüche 1 bis 7, **dadurch gekennzeichnet, dass** die Lösung ein Additiv enthält, welches die Bindung von aromatischen Verbindungen an Proteine weitgehend verhindert oder bereits gebundene aromatische Verbindungen wieder löst.

9. Verfahren nach Anspruch 8, **dadurch gekennzeichnet, dass** das Additiv eine hydrophobe oder amphiphile Substanz ist.

10. Verfahren nach Anspruch 9, **dadurch gekennzeichnet, dass** das Additiv ausgewählt ist aus der Gruppe bestehend aus Polyvinylalkoholen, Polyvinylpyrrolidonen, Tween 20, Tween 40, Tween 60, Tween 80, Nonidet P40, Polyethylenglycolen, Deoxycholat, CHAPS, oxyethyliertem Alkylphenol, partiell mit Fettsäure verestertem Polyoxyethylen, Polyoxyethylen-Fettalkoholderivaten, Dodecyl-N-betain, Palmitoyllyso-lecithin, Dodecyl-β-alanin, N-Dodecylaminoethansulfonsäure, Tetradecylammoniumbromid, Hexadecyltrimethylammoniumchlorid, Dodecylpyrimidiniumchlorid, Ethylenoxid-Propylenoxid Kondensaten, Cetyltrimethylammoniumbromid, Dodecyltrimethylbenzyl-ammoniumchlorid, oxyethylierten Aminen, sulfatiertem oxyethyliertem Alkylphenol, Natriumcholat, Natrium-desoxycholat, Natriumsulfoethylsulfonat, N-Methyl-N-oleylethanolsulfonat und Natriumdodecylbenzensulfonat.

11. Verfahren nach Anspruch 9, **dadurch gekennzeichnet, dass** das Additiv ein Polyvinylalkohol, Polyvinylpyrrolidon oder ein oxyethyliertes Alkylphenol ist.

12. Verfahren nach einem der Ansprüche 8 bis 11 **dadurch gekennzeichnet, dass** das Additiv gleichzeitig mit der aromatischen Verbindung aus der Lösung entfernt wird.

13. Verfahren nach Anspruch 1, **dadurch gekennzeichnet, dass** die Lösung Blutplasma oder ein Plasmaprodukt ist.

## Claims

1. A process for removing aromatic compounds from an aqueous solution which contains proteins or peptides having biological activity, with this solution being brought into contact with a support material which is modified with C₁ to C₅ alkyl radicals and the protein-containing or peptide-containing solution subsequently being separated from the modified support material.

2. The process as claimed in claim 1, wherein the aromatic compound is selected from the group consisting of: acridine, hypericin, psoralen, methylene blue and derivatives of these compounds.

3. The process as claimed in claim 1, wherein the aromatic compound is acridine and/or an acridine derivative.

4. The process as claimed in at least one of claims 1 to 3, wherein the support material is a silica gel.

5. The process as claimed in claim 4, wherein the silica gel is hydrophobically modified with ethyl groups.

6. The process as claimed in one of claims 1 to 5, wherein the process is carried out by means of column chromatography or batch chromatography.

7. The process as claimed in one of claims 1 to 5, wherein the process is carried out by filtration through the support material or flow over the support material.

8. The process as claimed in one of claims 1 to 7, wherein the solution contains an additive which substantially prevents the binding of aromatic compounds to proteins or releases aromatic compounds which have already been bound.

9. The process as claimed in claim 8, wherein the additive is a hydrophobic or amphiphilic substance.

10. The process as claimed in claim 9, wherein the additive is selected from the group consisting of polyvinyl alcohols, polyvinylpyrrolidones, Tween 20, Tween 40, Tween 60, Tween 80, Nonidet P40, polyethylene glycols, deoxycholate, CHAPS, ethoxylated alkylphenol, polyoxyethylene which is partially esterified with fatty acid, polyoxyethylene fatty alcohol derivatives, dodecyl-N-betaine, palmitoyllysolecithin, dodecyl-β-alanine, N-dodecylaminoethanesulfonic acid, tetradecylammonium bromide, hexadecyltrimethylammonium chloride, dodecylpyrimidinium chloride, ethylene oxide-propylene oxide condensates, cetyltrimethylammonium bromide, dodecyltrimethylbenzylammonium chloride, ethoxylated amines, sulfated ethoxylated alkylphenol, sodium cholate, sodium deoxycholate, sodium sulfoethylsulfonate, N-methyl-N-oleylethanolsulfonate and sodium dodecylbenzenesulfonate.

11. The process as claimed in claim 9, wherein the additive is a polyvinyl alcohol or polyvinylpyrrolidone, or an ethoxylated alkylphenol.

12. The process as claimed in one of claims 8 to 11, wherein the additive is removed from the solution at the same time as the aromatic compound.

13. The process as claimed in claim 1, wherein the solution is blood plasma or a plasma product.

## Revendications

1. Procédé pour éliminer les composés aromatiques d'une solution aqueuse, qui comprend des protéines ou des peptides ayant une activité biologique, cette solution étant mise en contact avec un matériau support qui est modifié par des radicaux alkyle en C₁ à C₅ et la solution contenant des protéines ou des peptides étant ensuite séparée du matériau support modifié.

2. Procédé selon la revendication 1, **caractérisé en ce que** le composé aromatique est choisi parmi le groupe constitué de :
l'acridine, l'hypericine, le psoralène, le bleu de méthylène et les dérivés de ces composés.

3. Procédé selon la revendication 1, **caractérisé en ce que** le composé aromatique est l'acridine ou un dérivé d'acridine.

4. Procédé selon l'une quelconque des revendications 1 à 3, **caractérisé en ce que** le matériau support est un gel de silice.

5. Procédé selon la revendication 4, **caractérisé en ce que** le gel de silice est modifié de manière hydrophobe avec des groupes éthyle.

6. Procédé selon l'une quelconque des revendications 1 à 5, **caractérisé en ce que** le procédé est réalisé par chromatographie sur colonne ou chromatographie de type batch.

7. Procédé selon l'une quelconque des revendications 1 à 5, **caractérisé en ce que** le procédé est réalisé par filtration à travers le matériau support ou par immersion du matériau support.

8. Procédé selon l'une quelconque des revendications 1 à 7, **caractérisé en ce que** la solution contient un additif qui inhibe substantiellement largement la liaison des composés aromatiques aux protéines ou libère les composés aromatiques déjà liés.

9. Procédé selon la revendication 8, **caractérisé en ce que** l'additif est une substance hydrophobe ou amphiphile.

10. Procédé selon la revendication 9, **caractérisé en ce que** l'additif est choisi parmi le groupe constitué des alcools polyvinyliques, polyvinylpyrrolidones, Tween 20, Tween 40, Tween 60, Tween 80, Nonidet P40, polyéthylèneglycols, le désoxycholate, CHAPS, l'alkylphénol oxyéthyléné, le polyoxyéthylène partiellement estérifié avec un acide gras, des dérivés d'alcool gras de polyoxyéthylène, la dodécyl-N-bétaïne, la lysolécithine de palmitoyle, la dodécyl-β-alanine, l'acide N-dodécylaminoéthanesulfonique, le bromure de tétradécylammonium, le chlorure d'hexadécyltriméthylammonium, le chlorure de dodécylpyrimidinium, les condensés d'oxyde d'éthylène / oxyde de propylène, le bromure de cétyltriméthylammonium, le chlorure de dodécyltriméthylbenzylammonium, les aminés oxyéthylénées, l'alkylphénol sulfaté oxyéthyléné, le cholate de sodium, le désoxycholate de sodium, le sulfoéthylsulfonate de sodium, le N-méthyl-N-oleyléthanolsulfonate et le dodécylbenzènesulfonate de sodium.

11. Procédé selon la revendication 9, **caractérisé en ce que** l'additif est un alcool polyvinylique, une polyvinylpyrrolidone ou un alkylphénol oxyéthyléné.

12. Procédé selon l'une quelconque des revendications 8 à 11, **caractérisé en ce que** l'additif est éliminé de la solution en même temps que composé aromatique.

13. Procédé selon la revendication 1, **caractérisé en ce que** la solution est un plasma sanguin ou un produit plasmique.
